# EUROPÄISCHE PATENTSCHRIFT

(11) **EP 0 590 459 B1**
(45) Veröffentlichungstag und Bekanntmachung des Hinweises auf die Patenterteilung: **23.07.1997**
(21) Anmeldenummer: 93115088.2
(22) Anmeldetag: 20.09.1993
(51) Int. Cl.: C07D 317/46

(54) **Verfahren zur Herstellung von Difluor- und Chlorfluorbenzodioxolen**
Process for the preparation of difluoro - and chlorofluorobenzodioxolanes
Procédé de préparation de difluoro- et de chlorofluorobenzodioxoles

(30) Priorität: 02.10.1992 DE 4233199
(43) Veröffentlichungstag der Anmeldung: 06.04.1994
(73) Patentinhaber: BAYER AG, 51368 Leverkusen (DE)
(72) Erfinder: Schnalke, Karl-Erwin, Dr., D-51375 Leverkusen (DE); Heinrich, Josef, Dr., D-42719 Solingen-Wald (DE); Döring, Fritz, D-51519 Odenthal-Glöbusch (DE)

(56) Entgegenhaltungen:
- EP-A- 0 008 732
- EP-A- 0 041 131

## Beschreibung

Die vorliegende Erfindung betrifft ein neues Verfahren zur Herstellung der bekannten Difluor- und Chlorfluorbenzodioxole, die als Zwischenprodukte für die Synthese hochaktiver Verbindungen auf den verschiedensten Gebieten verwendet werden können, z.B. zur Synthese von Wirkstoffen mit fungiziden Eigenschaften, geeignet zum Einsatz im Pflanzenschutz.

Es ist bereits bekannt, daß Difluorbenzodioxol aus der Dichlorverbindung mit Kaliumfluorid in Gegenwart eines Katalysators und gegebenenfalls in Gegenwart eines Lösungsmittels hergestellt werden kann (vgl. EP O 423009). Weiterhin ist die Synthese von aromatischen Verbindungen mit perfluorierten Seitenketten bekannt, deren Fluorierung mit Kaliumfluorid in Gegenwart von Lewissäuren durchgeführt wird (vgl. DE 3 315 147). Fluorsubstituierte Benzodioxole können aus den entsprechenden Dichlorverbindungen auch mit Ammonium- oder Alkalifluoriden in Gegenwart eines Katalysators und in Gegenwart eines Lösungsmittels hergestellt werden (vgl. DE 3 642 256).

All diesen Verfahren haften einige Nachteile an. Die Feststoffluorierungen mit Alkalimetallfluoriden arbeiten mit Lösungsmitteln, wodurch eine geringe Raum-Zeit-Ausbeute erzielt wird, ferner ist die Lösungsmittelrückgewinnung sehr aufwendig und es treten Entsorgungsprobleme mit den großen Mengen Alkalimetallchloriden auf.

Weiterhin ist ein Verfahren bekannt, das mit Flußsäure arbeitet. Es wird ein großer Überschuß an wasserfreier Flußsäure vorgelegt und das Dichlorbenzodioxol zugetropft (vgl. EP 41131). Diese Reaktion läuft zwar glatt ab, aber durch die thermische Belastung bei der destillativen Rückgewinnung der überschüssigen Flußsäure wird das Produkt wieder teilweise zerstört Außerdem gibt es bei der destillativen Reinigung des Produktes selbst erhebliche Probleme mit hochsiedenden Nebenprodukten, die Fluorwasserstoff abspalten und zu einer Verfärbung des Produktes führen. Außerdem wird natürlich auch die Ausbeute reduziert, sie liegt zwischen 68 und 78 % der Theorie.

Es wurde nun gefunden, daß die bekannten Benzodioxole der Formel (I) 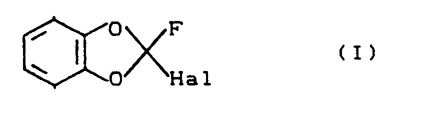 in welcher
Hal für Chlor oder Fluor steht,
erhalten werden, wenn man Dichlorbenzodioxol der Formel (II) 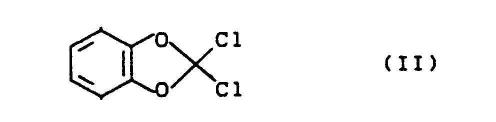 vorlegt und stöchiometrische Mengen (1 bzw. 2 Mol) wasserfreier Flußsäure bei Temperaturen zwischen -10°C und Raumtemperatur und einem Druck zwischen 0 und 25 bar zudosiert.

Es ist als ausgesprochen überraschend zu bezeichnen, daß sich die Benzodioxole der Formel (I) nach dem erfindungsgemäßen Verfahren in extrem hoher Ausbeute und ausgezeichneter Reinheit herstellen lassen. Aufgrund des bekannten Standes der Technik war nämlich nicht damit zu rechnen, daß die Verwendung von nur stöchiometrischen Mengen wasserfreier Flußsäure die gewünschten Benzodioxole in so hoher Selektivität liefert und die Fluorierung außerdem auch auf der Stufe des Chlorfluorbenzodioxols angehalten werden kann und ebenfalls so gute Ausbeuten liefert.

Das erfindungsgemäße Verfahren zeichnet sich durch eine Reihe von Vorteilen aus, So ermöglicht es, wie bereits erwähnt, die Herstellung von Difluorbenzodioxol in extrem hoher Ausbeute und ausgezeichneter Reinheit, ebenso die von Chlorfluorbenzodioxol in guten Ausbeuten.

Von besonderem Vorteil ist dabei, daß durch den stöchiometrischen Einsatz der wasserfreien Flußsäure die aufwendige Flußsäure-Rückgewinnung entfällt und eine sehr hohe Raum-Zeit-Ausbeute erzielt wird. Durch die hohe Selektivität wird ebenfalls die Aufarbeitung des Produktes sehr einfach, da kaum Rückstände anfallen, die entsorgt werden müssen.

Wir haben es also mit einem Verfahren zu tun, das auch im technischen Maßstab reibungslos durchgeführt werden kann, die Verbindungen auch dann in hohen Ausbeuten und großer Reinheit liefert und geringe Umweltbelastungen durch Destillationsrückstände, organisch belastete Salzrückstände gegeben sind.

Setzt man Dichlorbenzodioxol mit 1 bzw. 2 Mol wasserfreier Flußsäure um, so kann der Verlauf des erfindungsgemäßen Verfahrens durch das folgende Formelschema veranschaulicht werden:

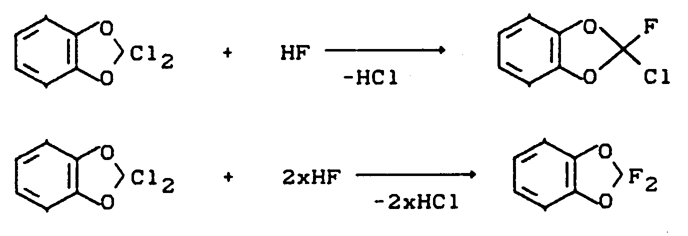

Das bei der Durchführung des erfindungsgemäßen Verfahrens als Ausgangsmaterial benötigte Dichlorbenzodioxol ist bekannt und kann nach bekannten Verfahren hergestellt werden (vgl. US 4 438 275 und die Referenzen in Spalte 15, H. Gross-Monatsberichte Deutsch. Akad. Wiss. Berlin 6(5), 356-362 (1964)). So kann es aus Benzodioxol hergestellt werden (vgl. J. Chem. Soc. 93, 566 (1988)), oder aus dem entsprechenden Katecholcarbonat (Chem. Ber. 96, 1382 (1963)) oder dem entsprechenden Katecholorthoformat (Chem. Ber. 94, 544 (1961)) durch Reaktion mit Phosphorpentachlorid. Das dabei einzusetzende Benzodioxol ist ein Kaufprodukt.

Bei der Durchführung des erfindungsgemäßen Verfahrens wird das Dichlorbenzodioxol vorgelegt und die wasserfreie Flußsäure unter Stickstoff innerhalb von 4 Stunden zudosiert, wobei die Abführung des Salzsäuregases der reaktionsbestimmende Schritt ist. Nach einer gewissen Nachreaktionszeit wird das Reaktionsgemisch durch Destillation im Vakuum aufgearbeitet. Im Verlaufe des Prozesses läßt sich die Reaktion auf der Stufe des Chlorfluorbenzodioxoles anhalten und dieses destillativ entfernen.

Die Reaktionstemperatur kann bei der Durchführung des erfindungsgemäßen Verfahrens innerhalb eines bestimmten Bereiches variiert werden. Man arbeitet bei Temperaturen zwischen -10°C und Raumtemperatur. Im allgemeinen wird die Reaktion bei 0°C gestartet, wo das Ausgangsmaterial noch flüssig ist, und erst im Laufe der Reaktion auf die angegebenen Temperaturen abgekühlt, um die Reaktionsmischung flüssig zu halten.

Die Benzodioxole der Formel (I) sind wertvolle Vorprodukte zur Herstellung von hochaktiven Verbindungen, die u.a. im Pflanzenschutz z.B. zur Bekämpfung von Pilzen eingesetzt werden (vgl. DE-A 4 107 398 und EP O 333661). Aus Difluorbenzodioxol wird entweder die in 4-Stellung bromierte oder metallierte Verbindung hergestellt, diese dann mit Acrylnitril zum entsprechenden Acrylnitril-Derivat und dann weiter mit Phenylsulfonylmethylisocyanid zu einem hochwirksamen 4-Cyano-pyrrol-Derivat umgesetzt. Geht man über die 4-Brom-Verbindung, so kann der Reaktionsablauf durch das folgende Formelschemata dargestellt werden:

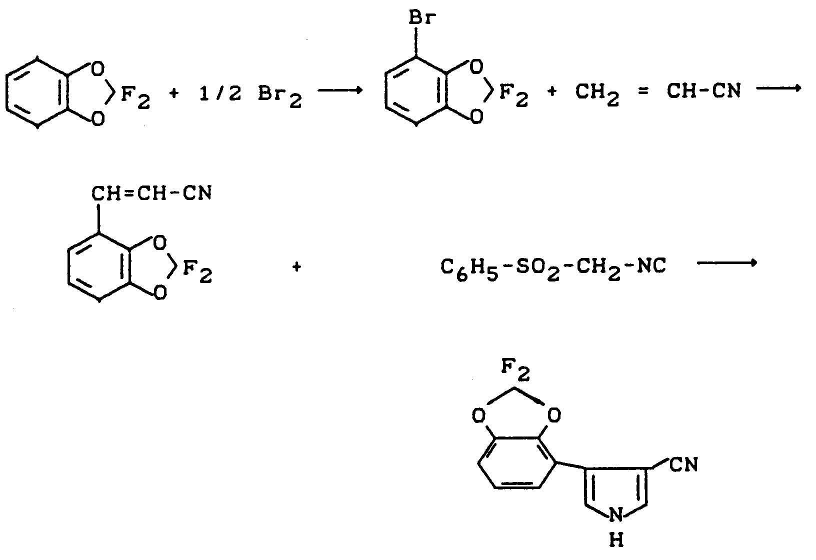

### Herstellungsbeispiele

### Beispiel 1

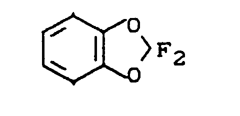

In einer Fluorierungsapparatur, bestehend aus einem 15 l Reaktor, einer 2 m langen Kolonne (Durchmesser 50 mm) gefüllt mit 6 mm VA Füllkörpern und Druckvorlagen werden 11 kg Dichlorbenzodioxol (Gehalt 92 %) bei 0°C vorgelegt. Der Entspannungsdruck der Anlage wird auf 6 bar eingestellt. Es werden 5 bar Stickstoff aufgedrückt. Über eine Dosierpumpe werden innerhalb von 4 Stunden bei einer Temperatur von 0°C bis -10°C 2200 g wasserfreier Flußsäure zudosiert. Das entstehende Salzsäure-Abgas wird bei einem Druck von 6 bar laufend entspannt. Es wird eine Stunde nachgerührt. Dabei wird die Temperatur auf 10°C angehoben. Anschließend wird der Anlagendruck innerhalb einer Stunde auf 1 bar entspannt.

Das Reaktionsgemisch wird im Vakuum destilliert. Man erhält bei 12 mbar/32 bis 34°C eine Auswaage von 7900 g mit einem Gehalt von 99,3 %. Das entspricht einer Ausbeute von 93,7% der Theorie.

### Beispiel 2

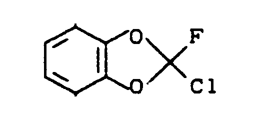

In der Apparatur und unter den Verfahrensbedingungen wie in Beispiel 1 angegeben werden 11 kg Dichlorbenzodioxol (92%ig) vorgelegt.

Bei 0°C bis -10°C werden 1240 g wasserfreier Flußsäure innerhalb von 4 Stunden zudosiert.

Nach einer Nachrührzeit von 0,5 Stunden wird der Anlagendruck entspannt und das Reaktionsgemisch destillativ aufgearbeitet.
Fraktion 1 Kp. 32 bis 36°C 15 mbar 1490 g
Fraktion 2 Kp. 36 bis 56°C 15 mbar 274 g
Fraktion 3 Kp. 56 bis 58°C 15 mbar 6846 g

Zusammensetzung der einzelnen Fraktionen:

| Fraktion | Difluorbenzodioxol in % | Chlorfluorbenzodioxol in % |
|---|---|---|
| 1 | 99,6 | 0,2 |
| 2 | 42,1 | 55,4 |
| 3 | - | 97,0 |

Das ergibt eine Ausbeute von 73,4 % Chlorfluorbenzodioxol mit einer Reinheit von >95 %.

## Patentansprüche

1. Verfahren zur Herstellung von Benzodioxolen der Formel (I) 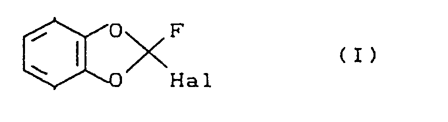 in welcher
Hai für ein Chlor oder Fluoratom steht, dadurch gekennzeichnet, daß man Dichlorbenzodioxol der Formel (II) 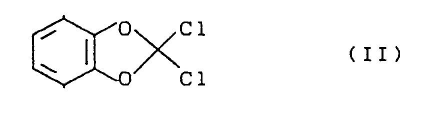 vorlegt und stächiometrische Mengen wasserfreier Flußsäure bei Temperaturen zwischen -10°C und Raumtemperatur und einem Druck zwischen 0 und 25 bar zudosiert.

2. Verfahren gemäß Anspruch 1, dadurch gekennzeichnet, daS man 2 Mol wasserfreier Flußsäure pro Mol Dichlorbenzodioxol einsetzt.

3. Verfahren gemäß Anspruch 1, dadurch gekennzeichnet, daß man 1 Mol wasserfreie Flußsäure pro Mol Dichlorbenzodioxol einsetzt.

## Claims

1. Process for the preparation of benzodioxoles of the formula (I) 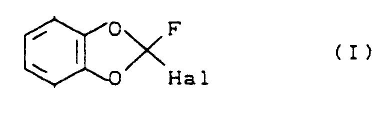 in which
Hal represents a chlorine atom or a fluorine atom, characterised in that dichlorobenzodioxole of the formula (II) 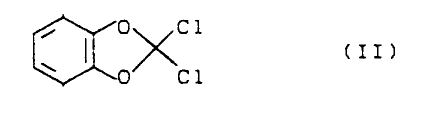 is initially introduced, and stoichiometric quantities of anhydrous hydrofluoric acid are metered in at temperatures between -10°C and room temperature and under a pressure of between 0 and 25 bar.

2. Process according to Claim 1, characterised in that 2 mol of anhydrous hydrofluoric acid are employed per mole of dichlorobenzodioxole.

3. Process according to Claim 1, characterised in that 1 mol of anhydrous hydrofluoric acid is employed per mole of dichlorobenzodioxole.

## Revendications

1. Procédé pour la préparation de benzodioxols répondant à la formule (I) 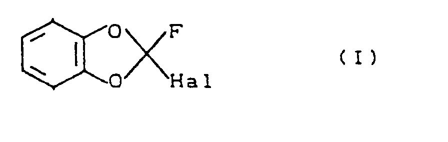 dans laquelle
Hal représente un atome de chlore ou un atome de fluor, caractérisé en ce qu'on dépose au préalable du dichlorobenzodioxol de formule (II) 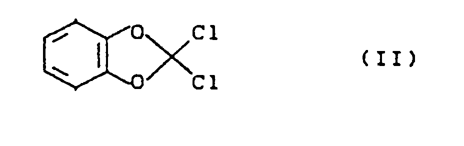 et on ajoute de manière dosée des quantités stoechiométriques d'acide fluorhydrique anhydre à des températures entre -10°C et la température ambiante et sous une pression entre 0 et 25 bar.

2. Procédé selon la revendication 1, caractérisé en ce qu'on met en oeuvre 2 moles d'acide fluorhydrique anhydre par mole de dichlorobenzodioxol.

3. Procédé selon la revendication 1, caractérisé en ce qu'on met en oeuvre 1 mole d'acide fluorhydrique anhydre par mole de dichlorobenzodioxol.
